# EUROPEAN PATENT SPECIFICATION

(11) **EP 3 358 960 B1**
(45) Date of publication and mention of the grant of the patent: **24.12.2025**
(21) Application number: 16854262.9
(22) Date of filing: 05.10.2016
(51) Int. Cl.: A23D 9/02, C11B 3/12

(54) **OIL COMPOSITIONS AND METHODS OF MAKING**
ÖLZUSAMMENSETZUNGEN UND VERFAHREN ZUR HERSTELLUNG
COMPOSITIONS D'HUILE ET LEURS PROCÉDÉS DE PRODUCTION

(30) Priority: 05.10.2015 US 201562237320 P
(43) Date of publication of application: 15.08.2018
(60) Divisional application: 25213261.8
(73) Proprietor: DSM IP Assets B.V., 6221 BE Maastricht (NL)
(72) Inventor: KRALOVEC, Jaroslav, Halifax, Nova Scotia B3S 1H4 (CA); LUIGART, Chris, Lexington, Kentucky 40502 (US); OXFORD, Mark, Eastern Passage, Nova Scotia B3G 0B2 (CA); PROPLESCH, Ralf, 4334 Sisseln (CH); REYES-SUAREZ, Erick, Columbia, Maryland 21045 (US); ROLLE, Alfred, Dartsmouth, Nova Scotia B3A 3V1 (CA); STEFANSKI, Michael Lenn, Winchester, Kentucky 40391 (US)
(74) Representative: Kraus & Lederer PartGmbB
(86) International application number: PCT/US2016/055599
(87) International publication number: WO 2017/062523

(56) References cited:
- WO-A1-01/51598
- WO-A1-2014/152283
- US-A- 4 792 418
- US-A- 5 130 061
- US-A- 5 215 630
- US-A1- 2013 196 008
- US-A1- 2014 030 377
- US-A1- 2014 128 465
- US-A1- 2014 335 580

## Description

### FIELD OF THE INVENTION

Disclosed herein are oil compositions that are enriched in polyunsaturated fatty acids; and methods of making the oil compositions according to claims 1 to 25. The oil is preferably a microbial or marine oil.

### BACKGROUND OF THE INVENTION

Fatty acids are classified based on the length and saturation characteristics of the carbon chain. Fatty acids are termed short chain, medium chain, or long chain fatty acids based on the number of carbons present in the chain, are termed saturated fatty acids when no double or triple bonds are present between the carbon atoms, and are termed unsaturated fatty acids when double or triple bonds are present. Unsaturated long chain fatty acids are monounsaturated when only one double or triple bond is present and are polyunsaturated when more than one double or triple bond is present.

Polyunsaturated fatty acids (PUFAs) are classified based on the position of the first double bond from the methyl end of the fatty acid; omega-3 (n-3) fatty acids contain a first double bond at the third carbon counting from the methyl terminal, while omega-6 (n-6) fatty acids contain a first double bond at the sixth carbon. For example, docosahexaenoic acid ("DHA") is an omega-3 long chain polyunsaturated fatty acid (LC-PUFA) with a chain length of 22 carbons and 6 double bonds, often designated as "22:6 n-3". Other omega-3 LC-PUFAs include eicosapentaenoic acid ("EPA"), designated as "20:5 n-3", and omega-3 docosapentaenoic acid ("DPA n-3"), designated as "22:5 n-3". Omega-6 LC-PUFAs include arachidonic acid ("ARA"), designated as "20:4 n-6", and omega-6 docosapentaenoic acid ("DPA n-6"), designated as "22:5 n-6".

Omega-3 fatty acids are biologically important molecules that affect cellular physiology due to their presence in cell membranes, regulate production and gene expression of biologically active compounds, and serve as biosynthetic substrates. Roche, H. M., Proc. Nutr. Soc. 58: 397-401 (1999). DHA, for example, accounts for approximately 15%-20% of the fatty acids making lipids in the human cerebral cortex, 30%-60% of the fatty acids making lipids in the retina, is concentrated in the testes and sperm, and is an important component of breast milk. Bergé, J.P., and Barnathan, G. Adv. Biochem. Eng. Biotechnol. 96:49-125 (2005). DHA accounts for up to 97% of the omega-3 fatty acids in the brain and up to 93% of the omega-3 fatty acids in the retina. Moreover, DHA is essential for both fetal and infant development as well as maintenance of cognitive functions in adults. *Id.* Because omega-3 fatty acids are not synthesized de novo in the human body, these fatty acids must be derived from nutritional sources. However, sources of omega-3 fatty acids can vary in the identity and amounts of LC-PUFAs produced. As such, a continuing need exists for omega-3 fatty acid sources having high amounts of LC-PUFAs with desirable LC-PUFA profiles and for oils that contain higher concentrations of LC-PUFAs. Previous concentration methods, as described for example in US 5,215,630, WO 01/51598, US 8,057,819, US 2014/0128465, and WO 2014/152283, have been shown to require numerous steps to achieve a desired concentration level, resulting in an inefficient process while often not obtaining the desired LC-PUFA content or profile. Other known methods are costly and time-consuming. Further, previous concentration methods have not provided a method of separation and concentration in one continuous process that provides a desired LC-PUFA content and profile. The inventors have surprisingly found a method of separation and concentration of oils comprising polyunsaturated fatty acids to produce desirable LC-PUFA profiles having higher concentrations of LC-PUF As that is less costly and time-consuming than previous methods.

### SUMMARY OF THE INVENTION

The present invention is directed to an oil comprising an ester fraction, wherein at least about 70% by weight of the fatty acids in the ester fraction is docosahexaenoic acid (DHA) and from about 0.5% to about 5% by weight of the fatty acids in the ester fraction is docosapentaenoic acid n-3 (DPA n-3) and the amount of DHA in ethyl ester form is at least about 65 % by weight of the total omega-3 fatty acids in the ester fraction; and from about 3% to about 13% by weight of the fatty acids in the ester fraction is DPA n3 in ethyl ester form and DPA n-6 in ethyl ester form and the amount of DHA in ethyl ester form is at least about 65% by weight of the total omega-3 fatty acids in the ester fraction; and wherein the oil has isomer value of not more than 1 wt%, wherein the isomer value is determined using gas chromatography measurement.. In some embodiments, less than about 5% by weight of the fatty acids in the ester fraction is eicosapentaenoic acid (EPA). In some embodiments, from about 0.1% to about 5% by weight of the fatty acids in the ester fraction is EPA. In some embodiments, from about 2% to about 8% by weight of the fatty acids in the ester fraction is DPA n-6. In some embodiments, the ester fraction comprises at least about 70% by weight of the oil.

The present disclosure is directed to an oil comprising an ester fraction, wherein at least about 70% by weight of the fatty acids in the ester fraction is docosahexaenoic acid (DHA) and from about 3% to about 13% by weight of the fatty acids in the ester fraction is docosapentaenoic acid n-3 (DPA n-3) and docosapentaenoic acid n-6 (DPA n-6). In some embodiments, from about 1% to about 5% by weight of the fatty acids in the ester fraction is DPA n-3. In some embodiments, from about 2% to about 8% by weight of the fatty acids in the ester fraction is DPA n-6. In some embodiments, less than about 5% by weight of the fatty acids in the ester fraction is eicosapentaenoic acid (EPA). In some embodiments, from about 0.1% to about 5% by weight of the fatty acids in the ester fraction is EPA. In some embodiments, the ester fraction comprises at least about 70% by weight of the oil.

The present disclosure is also directed to an oil comprising an ester fraction, wherein at least about 70% by weight of the fatty acids in the ester fraction is docosahexaenoic acid (DHA) and the amount of DHA in the ester fraction is at least about 65% by weight of the total omega-3 fatty acids in the ester fraction. In one embodiment, at least about 8% by weight of the fatty acids in the ester fraction is EPA. In some embodiments, the amount of EPA in the ester fraction is at least about 2% by weight of the total omega-3 fatty acids in the ester fraction.

The present disclosure is also directed to an oil comprising an ester fraction, wherein at least about 20% by weight of the fatty acids in the ester fraction is docosahexaenoic acid (DHA) and at least about 20% by weight of the fatty acids in the ester fraction is eicosapentaenoic acid (EPA). In some embodiments, from about 0.1% to about 5% by weight of the fatty acids in the ester fraction is DPA n-3.

The present disclosure is also directed to an oil comprising an ester fraction, wherein at least about 30% by weight of the fatty acids in the ester fraction is docosahexaenoic acid (DHA) and at least about 30% by weight of the fatty acids in the ester fraction is eicosapentaenoic acid (EPA). In some embodiments, from about 0.1% to about 5% by weight of the fatty acids in the ester fraction is DPA n-3.

The present disclosure is also directed to an oil comprising an ester fraction, wherein at least about 65% by weight of the fatty acids in the ester fraction is docosahexaenoic acid (DHA) and at least about 15% by weight of the fatty acids in the ester fraction is eicosapentaenoic acid (EPA). In some embodiments, from about 0.1% to about 5% by weight of the fatty acids in the ester fraction is DPA n-3.

The present disclosure is also directed to an oil comprising an ester fraction, wherein at least about 50% by weight of the fatty acids in the ester fraction is docosahexaenoic acid (DHA) and at least about 25% by weight of the fatty acids in the ester fraction is eicosapentaenoic acid (EPA). In some embodiments, from about 0.1% to about 5% by weight of the fatty acids in the ester fraction is DPA n-3.

The present invention is also directed to a process for separation and concentration of an oil comprising esters of polyunsaturated fatty acids, the process comprising subjecting the oil to at least one distillation step, wherein a first distillation step comprises feeding the oil to at least one apparatus and subjecting the oil to conditions to remove low-boiling compounds in a distillate.

In some embodiments, the oil is a microbial or marine oil.

In some embodiments, the oil is a microbial oil produced from a microorganism. In some embodiments, the microorganism is selected from the group comprising microalgae, bacteria, fungi and protists.

Also described is a food, supplement, or pharmaceutical composition comprising an oil of the invention.

### DETAILED DESCRIPTION OF THE INVENTION

The features and advantages of the invention may be more readily understood by those of ordinary skill in the art upon reading the following detailed description. It is to be appreciated that certain features of the invention that are, for clarity reasons, described above and below in the context of separate embodiments, may be combined to form sub- combinations thereof.

Embodiments identified herein as exemplary are intended to be illustrative.

In this specification and in the claims that follow, reference will be made to a number of terms, which shall be defined to have the following meanings:
The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein.

Disclosed herein are enriched oil compositions comprising polyunsaturated fatty acids; compositions containing the enriched oil compositions; and methods of making and using the enriched oil compositions.

Polyunsaturated fatty acids (PUFAs) are classified based on the position of the first double bond from the methyl end of the fatty acid; omega-3 (n-3) fatty acids contain a first double bond at the third carbon counting from the methyl end, while omega-6 (n-6) fatty acids contain a first double bond at the sixth carbon. For example, docosahexaenoic acid (DHA) is an omega-3 long chain polyunsaturated fatty acid (LC-PUFA) with a chain length of 22 carbons and 6 double bonds, often designated as "22:6n-3". In one embodiment, the PUFA is selected from an omega-3 fatty acid, an omega-6 fatty acid, and mixtures thereof. In another embodiment, the PUFA is selected from docosahexaenoic acid (DHA), eicosapentaenoic acid (EPA), docosapentaenoic acid (DPA), arachidonic acid (ARA), gamma-linolenic acid (GLA), dihomo-gamma-linolenic acid (DGLA), stearidonic acid (SDA), and mixtures thereof. In another embodiment, the PUFA is selected from DHA, DPA, EPA, and mixtures thereof. In another embodiment, the PUFA is DHA. In a further embodiment, the PUFA is DPA. In yet a further embodiment, the PUFA is EPA.

In some embodiments, the oil comprises at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% PUFA. In a preferred embodiment, the PUFA is in ester form. In a more preferred embodiment, the ester is an ethyl ester. In a preferred embodiment, the % by weight of the PUFA is the % by weight of the oil. In a more preferred embodiment, the % by weight of the PUFA is the % by weight of the fatty acids in an ester fraction.

In one embodiment, the oil comprises at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% by weight DHA. In a preferred embodiment, the DHA is in ester form. In a more preferred embodiment, the ester is an ethyl ester. In a preferred embodiment, the % by weight of DHA is the % by weight of the oil. In a more preferred embodiment, the % by weight of the DHA is the % by weight of the fatty acids in an ester fraction.

In some embodiments, the oil comprises about 4% to about 12%, about 5% to about 11%, about 6% to about 10%, or about 7% to about 9% DPA n-3 and DPA n-6. In a preferred embodiment, the DPA n-3 and DPA n-6 is in ester form. In a more preferred embodiment, the ester is an ethyl ester. In a preferred embodiment, the % by weight of the DPA n-3 and DPA n-6 is the % by weight of the oil. In a more preferred embodiment, the % by weight of the DPA n-3 and the DPA n-6 is the % by weight of the fatty acids in the ester fraction.

In some embodiments, the oil comprises from about 1% to about 5%, or about 3% to about 4% DPA n-3. In a preferred embodiment, the DPA n-3 is in ester form. In a more preferred embodiment, the ester is an ethyl ester. In a preferred embodiment, the % by weight of the DPA n-3 is the % by weight of the oil. In a more preferred embodiment, the % by weight of the DPA n-3 is the % by weight of the fatty acids in an ester fraction.

In some embodiments, the oil comprises about 2% to about 8%, about 3% to about 7%, or about 4% to about 6% DPA n-6. In a preferred embodiment, the DPA n-6 is in ester form. In a more preferred embodiment, the ester is an ethyl ester. In a preferred embodiment, the % by weight of the DPA n-6 is the % by weight of the oil. In a more preferred embodiment, the % by weight of the DPA n-6 is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% EPA. In a preferred embodiment, the EPA is in ester form. In a more preferred embodiment, the ester is an ethyl ester. In a preferred embodiment, the % by weight of the EPA is the % by weight of the oil. In a more preferred embodiment, the % by weight of the EPA is the % by weight of the fatty acids in an ester fraction.

In some embodiments, the oil comprises from about 0.1% to about 5%, about 0.5% to about 3%, or about 1% to about 2% EPA. In a preferred embodiment, the EPA is in ester form. In a more preferred embodiment, the ester is an ethyl ester. In a preferred embodiment, the % by weight of the EPA is the % by weight of the oil. In a more preferred embodiment, the % by weight of the EPA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% ARA. In a preferred embodiment, the ARA is in ester form. In a more preferred embodiment, the ester is an ethyl ester. In a preferred embodiment, the % by weight of the ARA is the % by weight of the oil. In a more preferred embodiment, the % by weight of the ARA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% GLA. In a preferred embodiment, the GLA is in ester form. In a more preferred embodiment, the ester is an ethyl ester. In a preferred embodiment, the % by weight of the GLA is the % by weight of the oil. In a more preferred embodiment, the % by weight of the GLA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% DGLA. In a preferred embodiment, the DGLA is in ester form. In a more preferred embodiment, the ester is an ethyl ester. In a preferred embodiment, the % by weight of the DGLA is the % by weight of the oil. In a more preferred embodiment, the % by weight of the DGLA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% SDA. In a preferred embodiment, the SDA is in ester form. In a more preferred embodiment, the ester is an ethyl ester. In a preferred embodiment, the % by weight of the SDA is the % by weight of the oil. In a preferred embodiment, the % by weight of the SDA is the % by weight of the fatty acids in an ester fraction.

In some embodiments, the comprises less than about 5%, less than about 4%, less than about 3%, less than about 2.5%, less than about 2%, less than about 2%, less than about 1.5%, less than about 1%, or less than about 0.5% of a polyunsaturated fatty acid having greater than 22 carbons (very long chain PUFAs). In some embodiments, the very long chain PUFA is 7,10,13,16,19,22,25 octacosaoctaenoic acid (C28:8). In a preferred embodiment, the oil comprises 0% 7,10,13,16,19,22,25 octacosaoctaenoic acid (C28:8). In a preferred embodiment, the very long chain PUFA is in ester form. In a more preferred embodiment, the ester is an ethyl ester. In a preferred embodiment, the % by weight of the very long chain PUFA is the % by weight of the oil. In a preferred embodiment, the % by weight of the very long chain PUFA is the % by weight of the fatty acids in an ester fraction.

In one embodiment, the oil comprises at least about 50%, at least about 55%, at least about 60%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% EPA. In a preferred embodiment, the EPA is in ester form. In a more preferred embodiment, the ester is an ethyl ester. In a preferred embodiment, the % by weight of the EPA is the % by weight of the oil. In a preferred embodiment, the % by weight of the EPA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% DHA. In a preferred embodiment, the DHA is in ester form. In a more preferred embodiment, the ester is an ethyl ester. In a preferred embodiment, the % by weight of the DHA is the % by weight of the oil. In a more preferred embodiment, the % by weight of the DHA is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% DPA n-3 and DPA n-6. In a preferred embodiment, the DPA n-3 and DPA n-6 is in ester form. In a more preferred embodiment, the ester is an ethyl ester. In a preferred embodiment, the % by weight of the DPA n-3 and DPA n-6 is the % by weight of the oil. In a preferred embodiment, the % by weight of the DPA n-3 and DPA n-6 is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% DPA n-3. In a preferred embodiment, the DPA n-3 is in ester form. In a more preferred embodiment, the ester is an ethyl ester. In a preferred embodiment, the % by weight of the DPA n-3 is the % by weight of the oil. In a more preferred embodiment, the % by weight of the DPA n-3 is the % by weight of the fatty acids in an ester fraction.

In some embodiments, the oil comprises less than about 5%, less than about 4%, less than about 3%, less than about 2.5%, less than about 2%, less than about 2%, less than about 1.5%, less than about 1%, or less than about 0.5% DPA n-6. In a preferred embodiment, the DPA n-6 is in ester form. In a more preferred embodiment, the ester is an ethyl ester. In a preferred embodiment, the % by weight of the DPA n-6 is the % by weight of the oil. In a preferred embodiment, the % by weight of the DPA n-6 is the % by weight of the fatty acids of the ester fraction.

In some embodiments, the oil comprises an ester fraction wherein at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% by weight of the fatty acids in the ester fraction is docosahexaenoic acid (DHA) and the amount of DHA in the ester fraction is at least about 65%, at least about 70%, at least about 75%, at least about 80%, at least about 85%, at least about 90% by weight of the total omega-3 fatty acids in the ester fraction. In some embodiments, at least about 8%, at least about 10%, at least about 15%, at least about 20%, at least about 35%, at least about 40% by weight of the fatty acids in the ester fraction is EPA. In some embodiments, the amount of EPA in the ester fraction is at least about 2%, at least about 3%, at least about 4%, at least about 5%, at least about 10%, at least about 15%, at least about 20%, at least about 25% by weight of the total omega-3 fatty acids in the ester fraction.

In some embodiments, the oil comprises an ester fraction of at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% by weight of the oil. In some embodiments, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99% by weight of the fatty acids in the ester fraction is DHA. In some embodiments, from about 0.5% to about 5%, about 1% to about 5%, or about 3% to about 4% by weight of the fatty acids in the ester fraction is DPA n-3.

In some embodiments, at least about 80%, at least about 85%, at least about 90%, at least about 91%, at least about 92%, at least about 93%, at least about 94%, at least about 95%, at least about 96%, at least about 97%, at least about 98% or at least about 99% by weight of the fatty acids in the ester fraction is DHA and DPA n-3.

In some embodiments, the DHA content of the fatty acids in the ester fraction is at least about 80%, at least about 85%, at least about 90%, at least about 91%, at least about 92%, at least about 93%, at least about 94%, at least about 95%, at least about 96%, at least about 97%, at least about 98% or at least about 99% by weight of the amount of DHA and DPA n-3 content of the fatty acids in the ester fraction.

In some embodiments, the DPA n-3 content of the fatty acids in the ester fraction is from about 0.5% to about 5%, about 1% to about 5%, or about 3% to about 4% of the DHA and DPA n-3 content of the fatty acids in the ester fraction.

In some embodiments, the DHA, DPA n-3 and DPA n-6 content of the fatty acids of the ester fraction is at least about 80%, at least about 85%, at least about 90%, at least about 91%, at least about 92%, at least about 93%, at least about 94%, at least about 95%, at least about 96%, at least about 97%, at least about 98% or at least about 99% of the total amount of fatty acids in the ester fraction.

In some embodiments, the DHA content of the fatty acids in the ester fraction is at least about 80%, at least about 85%, at least about 90%, at least about 91%, at least about 92%, at least about 93%, at least about 94%, at least about 95%, at least about 96%, at least about 97%, at least about 98% or at least about 99% of the DHA, DPA n-3 and DPA n-6 content of the fatty acids in the ester fraction.

In a preferred embodiment, the ester fraction is an ethyl ester.

In one embodiment, the DHA and EPA content of the fatty acids in the ester fraction is at least about 80%, at least about 85%, at least about 90%, at least about 91%, at least about 92%, at least about 93%, at least about 94%, at least about 95%, at least about 96%, at least about 97%, at least about 98% or at least about 99% of the total amount of fatty acids in the ester fraction. In some embodiments, the DHA content of the fatty acids in the ester fraction is an amount of at least about 80%, at least about 85%, at least about 90%, at least about 91%, at least about 92%, at least about 93%, at least about 94%, at least about 95%, at least about 96%, at least about 97%, at least about 98% or at least about 99% by weight of the total amount of fatty acids in the ester fraction. In some embodiments, the EPA content of the fatty acids in the ester fraction is less than about 5%, less than about 4%, less than about 3%, less than about 2.5%, less than about 2%, less than about 2%, less than about 1.5%, less than about 1%, or less than about 0.5% of the total amount of fatty acids in the ester fraction. In a preferred embodiment, the ester fraction is an ethyl ester.

In one embodiment, the DHA and EPA content of the fatty acids in the ester fraction is at least about 80%, at least about 85%, at least about 90%, at least about 91%, at least about 92%, at least about 93%, at least about 94%, at least about 95%, at least about 96%, at least about 97%, at least about 98% or at least about 99% of the total amount of fatty acids in the ester fraction. In some embodiments, the EPA content of the fatty acids in the ester fraction is an amount of at least about 80%, at least about 85%, at least about 90%, at least about 91%, at least about 92%, at least about 93%, at least about 94%, at least about 95%, at least about 96%, at least about 97%, at least about 98% or at least about 99% by weight of the total amount of fatty acids. In some embodiments, the DHA content of the fatty acids in the ester fraction is less than about 5%, less than about 4%, less than about 3%, less than about 2.5%, less than about 2%, less than about 2%, less than about 1.5%, less than about 1%, or less than about 0.5% if the total amount of fatty acids in the ester fraction. In a preferred embodiment, the ester fraction is an ethyl ester.

In some embodiments, the total isomer value of the oil is less than 5%, less than 4.5%, less than 4%, less than 3.5%, less than 3%, less than 2.5%, less than 2%, less than 1.5%, less than 1%, less than 0.5%, less than 0.1%, or 0%.

In some embodiments, the EPA isomer value of the oil is less than 5%, less than 4.5%, less than 4%, less than 3.5%, less than 3%, less than 2.5%, less than 2%, less than 1.5%, less than 1%, less than 0.5%, less than 0.1%, or 0%.

In some embodiments, the DHA isomer value of the oil is less than 5%, less than 4.5%, less than 4%, less than 3.5%, less than 3%, less than 2.5%, less than 2%, less than 1.5%, less than 1%, less than 0.5%, less than 0.1%, or 0%.

In some embodiments, the amount of DHA in the oil per gram of oil is from about 100 mg to about 300 mg, about 100 mg to about 600 mg, about 100 mg to about 800 mg, about 100 mg to about 900 mg, about 100 mg to about 950-mg, about 800 to about 950 mg, or 0 to about 100 mg.

In some embodiments, the amount of EPA in the oil per gram of oil is from about 100 mg to about 300 mg, about 100 mg to about 600 mg, about 100 mg to about 800 mg, about 100 mg to about 900 mg, about 100 mg to about 950-mg, about 800 to about 950 mg, or 0 to about 100 mg.

In some embodiments, the oil is a microbial or marine oil.

Oil produced by a microorganism or obtained from a microbial cell is referred to as "microbial oil". Oil produced by algae and/or fungi is referred to as an algal and/or a fungal oil, respectively.

As used herein, a "microorganism" refers to organisms such as algae, bacteria, fungi, protist, yeast, and combinations thereof, e.g., unicellular organisms. A microorganism includes but is not limited to, golden algae (e.g., microorganisms of the kingdom *Stramenopiles);* green algae; diatoms; dinoflagellates (e.g., microorganisms of the order *Dinophyceae* including members of the genus *Crypthecodinium* such as, for example, *Crypthecodinium cohnii* or *C*. *cohnii*); microalgae of the order Thraustochytriales; yeast (Ascomycetes or Basidiomycetes); and fungi of the genera *Mucor, Mortierella,* including but not limited to *Mortierella alpina* and *Mortierella sect. schmuckeri,* and *Pythium,* including but not limited to *Pythium insidiosum.*

In one embodiment, the microorganisms are from the genus *Mortierella,* genus *Crypthecodinium,* genus *Thraustochytrium,* and mixtures thereof. In a further embodiment, the microorganisms are from *Crypthecodinium Cohnii.* In a further embodiment, the microorganisms are *from Mortierella alpina.* In a still further embodiment, the microorganisms are from *Schizochytrium sp.* In yet an even further embodiment, the microorganisms are selected from *Crypthecodinium Cohnii, Mortierella alpina, Schizochytrium sp.,* and mixtures thereof.

In a still further embodiment, the microorganisms include, but are not limited to, microorganisms belonging to the genus *Mortierella,* genus *Conidiobolus,* genus *Pythium,* genus *Phytophthora,* genus *Penicillium,* genus *Cladosporium,* genus *Mucor,* genus *Fusarium,* genus *Aspergillus,* genus *Rhodotorula,* genus *Entomophthora,* genus *Echinosporangium,* and genus *Saprolegnia.*

In an even further embodiment, the microorganisms are from microalgae of the order Thraustochytriales, which includes, but is not limited to, the genera *Thraustochytrium* (species include *arudimentale, aureum, benthicola, globosum, kinnei, motivum, multirudimentale, pachydermum, proliferum, roseum, striatum*); the genera *Schizochytrium* (species include *aggregatum, limnaceum, mangrovei, minutum, octosporum);* the genera *Ulkenia* (species include *amoeboidea, kerguelensis, minuta, profunda, radiate, sailens, sarkariana, schizochytrops, visurgensis, yorkensis*); the *genera Aurantiacochytrium;* the genera *Oblongichytrium;* the genera *Sicyoidochytium;* the genera *Parientichytrium;* the genera *Botryochytrium;* and combinations thereof. Species described within Ulkenia will be considered to be members of the genus *Schizochytrium.* In another embodiment, the microorganisms are from the order Thraustochytriales. In yet another embodiment, the microorganisms are from *Thraustochytrium.* In still a further embodiment, the microorganisms are from *Schizochytrium sp.*

In certain embodiments, the oil can comprise a marine oil. Examples of suitable fish oils include, but are not limited to, Atlantic fish oil, Pacific fish oil, or Mediterranean fish oil, or any mixture or combination thereof. In more specific examples, a suitable fish oil can be, but is not limited to, pollack oil, bonito oil, pilchard oil, tilapia oil, tuna oil, sea bass oil, halibut oil, spearfish oil, barracuda oil, cod oil, menhaden oil, sardine oil, anchovy oil, capelin oil, herring oil, mackerel oil, salmonid oil, tuna oil, and shark oil, including any mixture or combination thereof. Other marine oils suitable for use herein include, but are not limited to, squid oil, cuttle fish oil, octopus oil, krill oil, seal oil, whale oil, and the like, including any mixture or combination thereof.

In some embodiments, a fatty acid as described herein can be a fatty acid ester or ester. In some embodiments, a fatty acid ester includes an ester of an omega-3 fatty acid, omega-6 fatty acid, and combinations thereof. In some embodiments, the fatty acid ester is a DHA ester, an EPA ester, or a combination thereof. In some embodiments, an oil or fraction thereof as described herein is esterified to produce an oil or fraction thereof comprising fatty acid esters. The term "ester" refers to the replacement of the hydrogen in the carboxylic acid group of the fatty acid molecule with another substituent. Examples of esters include methyl, ethyl, propyl, butyl, pentyl, t-butyl, benzyl, nitrobenzyl, methoxybenzyl, benzhydryl, and trichloroethyl. In some embodiments, the ester is a carboxylic acid protective ester group, esters with aralkyl (e.g., benzyl, phenethyl), esters with lower alkenyl (e.g., allyl, 2-butenyl), esters with lower-alkoxy-lower-alkyl (e.g., methoxymethyl, 2-methoxyethyl, 2-ethoxyethyl), esters with lower-alkanoyloxy-lower-alkyl (e.g., acetoxymethyl, pivaloyloxymethyl, 1- pivaloyloxyethyl), esters with lower-alkoxycarbonyl-lower-alkyl (e.g., methoxycarbonylmethyl, isopropoxycarbonylmethyl), esters with carboxy-lower alkyl (e.g., carboxymethyl), esters with lower-alkoxycarbonyloxy-lower-alkyl (e.g., 1- (ethoxycarbonyloxy)ethyl, 1-(cyclohexyloxycarbonyloxy)ethyl), esters with carbamoyloxy- lower alkyl (e.g., carbamoyloxymethyl), and the like. In some embodiments, the added substituent is a linear or cyclic hydrocarbon group, e.g., a C1-C6 alkyl, C1-C6 cycloalkyl, C1-C6 alkenyl, or C1-C6 aryl ester. In some embodiments, the ester is an alkyl ester, e.g., a methyl ester, ethyl ester or propyl ester. In some embodiments, the ester substituent is added to the free fatty acid molecule when the fatty acid is in a purified or semi-purified state.

Fatty acid esters, in particular polyunsaturated fatty acid esters, can be made in ways that are known to one of ordinary skill in the art.

For example, tri-acyl glycerides, di-acyl glycerides, and /or mono-acyl glycerides that contain fatty acids, particularly poly-unsaturated fatty acids, can be reacted with an alcohol in the presence of an acid or a base to produce esters. The disclosure of U.S. Patent Application No. 12/163,555, that published as U.S. Patent Application Pre-Grant Publication No. 2009/0023808, is incorporated by reference herein in its entirety.

Alcohols can include, for example, C₁-C₆ alkyl alcohols, for example, ethanol, methanol, n-propanol, iso-propanol, n-butanol, iso-butanol, sec-butanol, tert-butanol, n-pentanol, and n-hexanol. The alcohol can be used as the reaction solvent and co-reactant, either alone or with a co-solvent. The amount of the alcohol can range from 25 % to 50 % by weight of the reaction mixture, including all values and subranges therebetween as if explicitly written out. For example, the amount of alcohol in the reaction mixture can be 30%, 35%, 40% or 45% by weight of the reaction mixture.

The base can be, for example, a metal alkyloxide. Metal alkyloxides include sodium ethoxide, sodium methoxide, sodium n-propoxide, sodium iso-propoxide, sodium n-butoxide, sodium iso-butoxide, sodium sec-butoxide, sodium, tert-butoxide, sodium n-pentoxide, sodium n-hexoxide, lithium ethoxide, lithium methoxide, lithium n-propoxide, lithium iso-propoxide, lithium n-butoxide, lithium iso-butoxide, lithium sec-butoxide, lithium, tert-butoxide, lithium n-pentoxide, lithium n-hexoxide, potassium ethoxide, potassium methoxide, potassium n-propoxide, potassium iso-propoxide, potassium n- butoxide, potassium iso-butoxide, potassium sec-butoxide, potassium, tert-butoxide, potassium n-pentoxide, and /or potassium n-hexoxide.

In some situations, the base can be made by adding sodium metal, potassium metal, or lithium metal to an alcoholic solution.

In some situations, the base can be made by adding a metal hydride, such as lithium hydride, sodium hydride, or potassium hydride, to an alcoholic solution.

The ratio of base to oil, on a weight: weight basis can, for example, range from 1:1 to 1000:1, including all values and subranges therebetween as if explicitly written out. For example, the ratio of base to oil, on a weight to weight basis, can be 2:1, 3:1, 4:1, 5:1, 6:1, 7:1, 8:1, 9:1, 10:1, 20:1, 30:1, 40:1, 50:1, 60:1, 70:1, 80:1, 90:1, 100:1, 200:1, 300:1, 400:1, 500;1, 600:1, 700:1, 800:1, or 900:1.

The esterification reaction can be run at a temperature ranging from 10 °C to 100 °C, including all values and subranges therebetween as if explicitly written out. For example, the esterification reaction can be run at 20 °C, 30 °C, 40 °C, 50 °C, 60 °C, 70 °C, 80 °C, or 90 °C.

The esterification reaction can be run open to the atmosphere, or under an inert atmosphere such as nitrogen or argon.

Workup and isolation of the fatty acid esters can be done in ways known to one of ordinary skill, for example, by extraction with an organic solvent and / or water. The organic solvent can be, for example, pentane, hexane, di-ethyl ether, ethyl acetate, or a combination of these. The water can optionally contain other substances such as sodium bicarbonate, sodium carbonate, ammonium chloride and / or dilute mineral acid.

In some embodiments, the oil is transesterified to convert at least part of the ester fraction in the oil to a triglyceride fraction. Transesterification, in particular transesterification of polyunsaturated fatty acid esters, can be made in ways that are known to one of ordinary skill in the art.

In the present invention, any concentrating, reacting, and/or purifying technique can be combined with any other concentrating, reacting, and/or purifying technique to produce microbial oils enriched in: polyunsaturated fatty acids, their esters, their salts, aldehydes thereof and/or alcohols thereof. The enrichment techniques can be used in any order and combination.

In some embodiments, the present invention is a food, supplement, or pharmaceutical composition comprising an oil of the invention. The pharmaceutical composition can contain a pharmaceutically acceptable carrier.

In some embodiments, the composition is a food product. A food product is any food for non-human animal or human consumption, and includes both solid and liquid compositions. A food product can be an additive to animal or human foods. Foods include, but are not limited to, common foods; liquid products, including milks, beverages, therapeutic drinks, and nutritional drinks; functional foods; supplements; nutraceuticals; infant formulas, including formulas for pre-mature infants; foods for pregnant or nursing women; foods for adults; geriatric foods; and animal foods.

In some embodiments, the composition is an animal feed. An "animal" includes non-human organisms belonging to the kingdom Animalia, and includes, without limitation, aquatic animals and terrestrial animals. The term "animal feed" or "animal food" refers to any food intended for non-human animals, whether for fish; commercial fish; ornamental fish; fish larvae; bivalves; mollusks; crustaceans; shellfish; shrimp; larval shrimp; artemia; rotifers; brine shrimp; filter feeders; amphibians; reptiles; mammals; domestic animals; farm animals; zoo animals; sport animals; breeding stock; racing animals; show animals; heirloom animals; rare or endangered animals; companion animals; pet animals such as dogs, cats, guinea pigs, rabbits, rats, mice, or horses; primates such as monkeys (e.g., cebus, rhesus, African green, patas, cynomolgus, and cercopithecus), apes, orangutans, baboons, gibbons, and chimpanzees; canids such as dogs and wolves; felids such as cats, lions, and tigers; equids such as horses, donkeys, and zebras; food animals such as cows, cattle, pigs, and sheep; ungulates such as deer and giraffes; or rodents such as mice, rats, hamsters and guinea pigs; and so on. An animal feed includes, but is not limited to, an aquaculture feed, a domestic animal feed including pet feed, a zoological animal feed, a work animal feed, a livestock feed, and combinations thereof.

In some embodiments, the composition is a feed or feed supplement for any animal whose meat or products are consumed by humans, such as any animal from which meat, eggs, or milk is derived for human consumption. When fed to such animals, nutrients such as LC-PUFAs can be incorporated into the flesh, milk, eggs or other products of such animals to increase their content of these nutrients.

The present invention is also directed to a process for separation and concentration of an oil comprising esters of polyunsaturated fatty acids to produce an oil. The process comprises subjecting the oil to at least one distillation step, said at least one distillation step comprising a first and second distillation step, wherein the first distillation step comprises feeding the oil to at least on apparatus and subjecting the oil to conditions comprising heating to remove low-boiling compounds in a distillate and wherein the second distillation step comprises subjecting the oil to conditions to separate at least a portion of the long-chain polyunsaturated fatty acid (LC-PUFA) which is eicosapentaenoic acid (EPA) from at least a portion of a second LC-PUFA which is docosahexaenoic acid (DHA), wherein said at least one apparatus comprises a fractionating column that is attached to a wiped-film evaporator, wherein at least a portion of eicosapentaenoic acid (EPA) is removed in the distillate of the first distillation step, and wherein the obtained oil has an isomer value of not more than 1% by weight, wherein the isomer value is determined using gas chromatography measurement, wherein the temperature during said at least one distillation step is less than about 275°C.

In some embodiments, the process comprises subjecting the oil to at least one distillation step, wherein a first distillation step comprises feeding the oil to at least one apparatus and subjecting the oil to conditions to remove low-boiling compounds in a distillate. In a preferred embodiment, at least a portion of eicosapentaenoic acid (EPA) is removed in the distillate. In some embodiments, at least about 5%, at least about 10%, at least about 15%, at least about 20% of EPA is in the distillate of the first distillation step.

In some embodiments, at least about 50%, at least about 55%, at least about 60%, at least about 65%, at least about 70%, at least about 75% of one long-chain polyunsaturated fatty acid (LC-PUFA) is separated from a second LC-PUFA in the second distillation step. In a preferred embodiment, one LC-PUFA is EPA. In a preferred embodiment, the second LC-PUFA is DHA. In a more preferred embodiment, one LC-PUFA is EPA and the second LC-PUFA is DHA.

In some embodiments, at least about 50%, at least about 55%, at least about 60%, at least about 65%, at least about 70%, at least about 75% of one long-chain polyunsaturated fatty acid (LC-PUFA) is separated from a second LC-PUFA in the third distillation step. In a preferred embodiment, one LC-PUFA is EPA. In a preferred embodiment, the second LC-PUFA is DHA. In a more preferred embodiment, one LC-PUFA is EPA and the second LC-PUFA is DHA.

In some embodiments, the process comprises short path distillation, fractional distillation, falling-film evaporator, wiped-film evaporator, or combinations thereof. In a preferred embodiment, the process comprises fractional distillation.

In some embodiments, the apparatus is a fractionating column.

In some embodiments, the column comprises a rectification section. In some embodiments, the column has at least about 1 meter, at least about 2 meter, or at least about 3 meter of structured packing, having at least about 3-4, at least about 4-5, at least about 5-6, at least about 6-7, at least about 7-8, at least about 8-9, at least about 9-10, at least about 10-11, at least about 11-12, or at least about 12-13 theoretical stages.

In some embodiments, the column is attached to a vacuum. In some embodiments, the pressure at the top of the column is less than about 4 mbar, less than about 3.5 mbar, less than about 3 mbar, less than about 2.5 mbar, less than about 2 mbar, or less than about 1.5 mbar. In some embodiments, the pressure drop of the column is less than about 10 mbar, less than about 9 mbar, less than about 8 mbar, less than about 7 mbar, less than about 6 mbar, less than about 5 mbar, less than about 4 mbar, or less than about 3 mbar.

In some embodiments, the apparatus is a fractionating column or short-path distillation. In a preferred embodiment, the fractionating column is attached to a wiped-film evaporator. In some embodiments comprising more than one distillation step, the apparatus of each distillation step may be the same apparatus or may be in series.

In some embodiments, feeding the oil to at least one apparatus comprises a mid-column feed. In some embodiments, feeding the oil to at least one apparatus comprises an evaporator feed.

In a preferred embodiment, the oil subjected to a first distillation step is separated at the residue end and the distillate end in an amount of at least about 30%, at least about 40%, at least about 50%, at least about 60%, at least about 70%, or at least about 80% by weight. In a preferred embodiment, the oil collected at the residue end comprises at least about 10%, at least about 20%, at least about 30%, at least about 40%, at least about 50%, at least about 60%, at least about 70%, at least about 80%, at least about 85%, or at least about 90% by weight EPA and DHA. In a preferred embodiment, the oil collected at the distillate end comprises at least about 10%, at least about 20%, at least about 30%, at least about 40%, at least about 50%, at least about 60%, at least about 70%, at least about 80%, at least about 85%, or at least about 90% by weight polyunsaturated fatty acids having less than 20 carbons.

In a preferred embodiment, the oil subjected to the second distillation step is separated at the residue end and the distillate end in an amount of at least about 30%, at least about 40%, at least about 50%, at least about 60%, at least about 70%, or at least about 80% by weight. In a preferred embodiment, the oil collected at the residue end comprises at least about 10%, at least about 20%, at least about 30%, at least about 40%, at least about 50%, at least about 60%, at least about 70%, at least about 80%, at least about 85%, or at least about 90% by weight EPA. In a preferred embodiment, the oil collected at the distillate end comprises at least about 10%, at least about 20%, at least about 30%, at least about 40%, at least about 50%, at least about 60%, at least about 70%, at least about 80%, at least about 85%, or at least about 90% by weight DHA.

In a preferred embodiment, the oil subjected to the third distillation step is separated at the residue end and the distillate end in an amount of at least about 30%, at least about 40%, at least about 50%, at least about 60%, at least about 70%, or at least about 80% by weight. In a preferred embodiment, the oil collected at the residue end comprises at least about 10%, at least about 20%, at least about 30%, at least about 40%, at least about 50%, at least about 60%, at least about 70%, at least about 80%, at least about 85%, or at least about 90% by weight EPA. In a preferred embodiment, the oil collected at the distillate end comprises at least about 10%, at least about 20%, at least about 30%, at least about 40%, at least about 50%, at least about 60%, at least about 70%, at least about 80%, at least about 85%, or at least about 90% by weight DHA.

In some embodiments, the at least one distillation step comprises heating wherein the temperature less than about 250 °C, less than about 225 °C, less than about 200 °C, less than about 190 °C, less than about 180 °C, or less than about 170 °C, from about 100 ° C to about 250 °C, from about 125 ° C to 225 °C, from about 150 ° C to 200 °C, or from about 160 ° C to 190 °C.

In some embodiments, the reflux ratio is less than about 5, less than about 4.5, less than about 4, less than about 3.5, less than about 3, less than about 2.5, less than about 2, less than about 1.5, less than about 1, or less than about 0.5.

The present invention is also directed to an oil produced by any of the processes disclosed herein.

### EXAMPLES

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations that are apparent to one skilled in the art.

Analytical Methods: Determination of fatty acid compositions was performed using gas chromatograph with a flame ionization detector (GD/FID) according to European Pharmacopoeia method 2.04.29.

### Example 1

A crude oil was produced through fermentation of *Schizochytrium sp.* Table 1 shows the fatty acid profile of the starting oil.

**Table 1: Fatty Acid Profile-Starting Oil**

| **Fatty Acid** | **Area Percent (%)** |
|---|---|
| C14:0 | 1.3 |
| C16:0 | 18.3 |
| C18:0 | 1.1 |
| C18:1 n-9 | 2.1 |
| C20:4 n-6 | 1.3 |
| C20:5 n-3 (EPA) | 9.5 |
| C22:5 n-6 | 4.0 |
| C22:5 n-3 | 1.2 |
| C22:6 n-3 (DHA) | 58.1 |

*Transesterification:* To convert the starting oil from a triglyceride to an ethyl ester, a one liter round-bottomed flask was charged with 400 g of the dry, crude oil and a solution of sodium ethoxide (3.2 g) dissolved in ethanol (120 g) was added. The mixture was heated to 75°C while stirring under a N₂ atmosphere and then maintained at this temperature for 1 hour. The reaction mixture was allowed to cool to 30°C, transferred to a separatory funnel, and the bottom glycerol layer was drained. The top oil layer was then transferred to a clean 1L round- bottomed flask and a solution of sodium ethoxide (0.32 g) in ethanol (12.0 g) was added. The mixture was again heated to 75°C while stirring under a N₂ atmosphere and then maintained at this temperature for 1 hour. The ethanol was removed using a rotary evaporator, the residue was washed with a citric acid solution (1% w/w) until the pH of the aqueous washing portions was no longer basic, washed with water, and dried under vacuum.

Analytical Methods: Determination of oligomers and lipid class was performed using size exclusion chromatography with refractive index detection (SEC/RI). Polymer based Tosahaas TSK-GEL were used for gel permeation chromatography. The apparatus was assembled with two TSK-GEL columns + a guard column and equilibrated with tetrahydrofuran (THF) at a flow rate of 0.600 mL/min. The oil samples were dissolved in THF at a concentration of 15 mg/mL and injected into the chromatorgraph in 10.0µL injection volume. Table 2 shows the lipid class profile of the starting oil after transesterification.

**Table 2: Lipid Class-Starting Oil**

| **Lipid Class** | % |
|---|---|
| Ethyl Ester | 96 |
| Monoglyceride | 2.6 |
| Diglyceride | 1.2 |
| Triglyceride | 0.1 |

*Purification and Concentration, Trial 1:* The oil was fractionated via three passes through a short path distillation apparatus using a temperature range from 120°C to 180°C and vacuum of approximately 20mtorr. On the first pass, the starting oil was passed through the short path distillation apparatus. The distillate contained 110 mg/g EPA and 322 mg/g DHA (Sample 1D) and the residue contained 70 mg/g EPA and 759 mg/g DHA (Sample 1R). On the second pass, the residue from the first pass was fed through the short path distillation apparatus, resulting in 72 mg/g EPA and 789 mg/g DHA in the distillate (Sample 2D). The residue could not be sampled due to the viscosity (Sample 2R). On the third pass, the distillate from the second pass was fed through the short path distillation apparatus, resulting in 126 mg/g EPA and 715 mg/g DHA in the distillate (Sample 3D) and 43 mg/g EPA and 811 mg/g DHA residue in the residue (Sample 3R). Results are shown in Table 3.

**Table 3: Short path Distillation Results, Trial 1**

| **Sample** | **EPA (mg/g)** | **DHA (mg/g)** | **Total Omega-3 (mg/g)** |
|---|---|---|---|
| Starting oil | 87 | 548 | 646 |
| First Pass-Distillate (Sample 1D) | 110 | 322 | 451 |
| First Pass-Residue (Sample 1R) | 70 | 759 | 853 |
| Second Pass-Distillate (Sample 2D) | 72 | 789 | 887 |
| Second Pass-Residue (Sample 2R) | ---- | ---- | ---- |
| Third Pass-Distillate (Sample 3D) | 126 | 715 | 868 |
| Third Pass-Residue (Sample 3R) | 43 | 811 | 877 |

*Purification and Concentration, Trial 2:* The oil was fractionated via four passes through a short path distillation apparatus using a temperature range from 120°C to 180°C and vacuum of approximately 20mtorr. On the first pass, the starting oil was passed through the short path distillation apparatus. The distillate contained 69 mg/g EPA and 154 mg/g DHA (Sample 1D) and the residue contained 94 mg/g EPA and 641 mg/g DHA (Sample 1R). On the second pass, the residue from the first pass was fed through the short path distillation apparatus, resulting in 131 mg/g EPA and 373 mg/g DHA in the distillate (Sample 2D) and 84 mg/g EPA and 717 mg/g DHA in the residue (Sample 2R). On the third pass, the residue from the second pass was fed through the short path distillation apparatus, resulting in 86 mg/g EPA and 742 mg/g DHA in the distillate (Sample 3D). The residue could not be sampled due to the viscosity (Sample 3R). On the fourth pass, the distillate from the third pass was fed through the short path distillation apparatus, resulting in 117 mg/g EPA and 722 mg/g DHA in the distillate (Sample 4D) and 26 mg/g EPA and 785 mg/g DHA in the residue (Sample 4R). Results are shown in Table 4.

**Table 4: Short path Distillation Results, Trial 2**

| **Sample** | **EPA (mg/g)** | **DHA (mg/g)** | **Total Omega-3 (mg/g)** |
|---|---|---|---|
| Starting oil | 87 | 548 | 646 |
| First Pass-Distillate (Sample 1D) | 69 | 154 | 234 |
| First Pass-Residue (Sample 1R) | 94 | 641 | 758 |
| Second Pass-Distillate (Sample 2D) | 131 | 373 | 527 |
| Second Pass-Residue (Sample 2R) | 84 | 717 | 825 |
| Third Pass-Distillate (Sample 3D) | 86 | 742 | 853 |
| Third Pass-Residue (Sample 3R) | ---- | ---- | ---- |
| Fourth Pass-Distillate (Sample 4D) | 117 | 722 | 867 |
| Fourth Pass-Residue (Sample 4R) | 26 | 785 | 832 |

*Purification and Concentration, Trial 3:* A different starting oil was used for this example than in Trials 1 and 2. The oil was produced through fermentation of *Schizochytrium sp.* and was subjected to the same transesterification process as above. The oil was fractionated via three passes through a short path distillation apparatus using a temperature range from 120°C to 180°C and vacuum of approximately 20mtorr. On the first pass, the starting oil was passed through a short path distillation apparatus. The distillate contained 112 mg/g EPA and 332 mg/g DHA (Sample 1D) and the residue contained 60 mg/g EPA and 733 mg/g DHA (Sample 1R). On the second pass, the residue from the first pass was fed through the short path distillation apparatus, resulting in 76 mg/g EPA and 766 mg/g DHA in the distillate (Sample 2D). The residue could not be sampled due to the viscosity (Sample 2R). On the third pass, the distillate from the second pass was fed through the short path distillation apparatus, resulting in 96 mg/g EPA and 786 mg/g DHA in the distillate (Sample 3D) and 22 mg/g EPA and 805 mg/g DHA residue in the residue (Sample 3R). Results are shown in Table 5.

**Table 5: Short path Distillation Results, Trial 3**

| **Sample** | **EPA (mg/g)** | **DHA (mg/g)** | **Total Omega-3 (mg/g)** |
|---|---|---|---|
| Starting oil | 86 | 525 | 630 |
| First Pass-Distillate (Sample 1D) | 112 | 332 | 462 |
| First Pass-Residue (Sample 1R) | 60 | 733 | 815 |
| Second Pass-Distillate (Sample 2D) | 62 | 766 | 851 |
| Second Pass-Residue (Sample 2R) | ---- | ---- | ---- |
| Third Pass-Distillate (Sample 3D) | 96 | 786 | 907 |
| Third Pass-Residue (Sample 3R) | 22 | 805 | 848 |

### Example 2

A crude oil was produced through fermentation of *Schizochytrium sp.*

*Transesterification:* A one liter round-bottomed flask was charged with 400 g of the dry, filtered, crude oil and a solution of sodium ethoxide (3.2 g) dissolved in ethanol (120 g) was added. The mixture was heated to 75°C while stirring under a N₂ atmosphere and then maintained at this temperature for 1 hour. The reaction mixture was allowed to cool to 30°C, transferred to a separatory funnel and the bottom glycerol layer was drained. The top oil layer was then transferred to a clean 1L round- bottomed flask and a solution of sodium hydroxide (0.32 g) in ethanol (12.0 g) was added. The mixture was again heated to 75°C while stirring under a N₂ atmosphere and then maintained at this temperature for 1 hour. The ethanol was removed using a rotary evaporator, washed with a citric acid solution (1% w/w) until the pH of the aqueous washing portions was no longer basic, washed with water, and dried under vacuum.

*Purification and Concentration:* The oil was fractionated at a temperature not exceeding 210°C with a pressure drop of the column of 2.3 mbar. The heavy fraction was further refined using short path distillation at a pressure of 0.01 mbar and a temperature of 140°C. The DHA concentrate was isolated in the amount of 225 mg and purity of 890 mg/g.

### Example 3

A crude fish oil was subjected to a separation and concentration process.

*Transesterification:* An esterification reaction between the purified marine oil and ethyl alcohol, using sodium ethoxide as a catalyst, was used to produce a marine oil ethyl ester. Ethanol and sodium ethoxide was added to a purified marine oil that had been heated. The glycerol was separated from the marine oil. Ethanol and sodium ethoxide was then added again to the marine oil (after removal of the glycerol). The ethyl ester marine oil formed was centrifuged for further glycerol removal. The sodium ethoxide was neutralized by 5% addition by weight of the marine oil of a 9% citric acid solution. The acid wash layer was separated from the ethyl ester marine oil, water was added, and the ethyl ester marine oil was dried.

The ethyl ester marine oils used in Examples 4-9 were prepared as in this Example 3.

### Example 4

A crude fish oil was subjected to a separation and concentration process. A falling-film evaporator with a rectification column followed by short path distillation (SPD) was evaluated. The column contained approximately 1 meter of packing having approximately 3-4 theoretical stages. An ethyl ester marine oil was fed through the recycle stream at a temperature of 205°C. Concentration results are shown in Table 7. Isomer results are shown in Table 8.

Analytical Methods: Isomers and related impurities were measured using gas chromatography. About 25 mg of sample was weighed and dissolved in 25 mL of hexanes. A silver ion SPE cartridge was mounted on the manifold; the cartridge was conditioned with 4 mL of acetone and equilibrated with 4 mL of hexane. 1 mL of the sample solution was loaded onto the cartridge and pulled through. The cartridge was washed with 6 mL of acetone. Into a new 15 mL test tube, the sample was rinsed from the cartridge with 2 mL of acetone/acetonitrile (3:2). The test tube was removed from the manifold and solvent blown under a nitrogen stream. 1 mL of hexane was added to the test tube. The solution was transferred to a GC vial. An Agilent DB Wax 30m X 0.25mm X 0.25µm column using Helium @ 1 mL/min was used. The injection volume was 1 µL and the detector was FID @ 260°C. The gradient was 100°C for 1.0 min, ramp @ 20/min to 210°C, hold for 40 minutes.

**Table 7: Column Followed by Short Path Distillation, 1m packing, EPA/DHA concentration**

| **Sample** | **EPA (mg/g)** | **DHA (mg/g)** |
|---|---|---|
| Starting oil | 176 | 85 |
| Column-Distillate | 70.7 | 0.3 |
| Column-Residue | 323.4 | 191.7 |
| SPD-Distillate | 378 | 210.2 |
| SPD-Residue | 19.7 | 21.9 |

**Table 8: Column Followed by Short Path Distillation, 1m packing, Isomers**

| **Sample** | **EPA Isomers (%)** | **DHA Isomers (%)** | **Total Isomers (%)** |
|---|---|---|---|
| Starting oil | 0.3 | 0.8 | 0.5 |
| Column-Residue | 0.4 | 2.6 | 2.0 |
| SPD-Distillate | 2.6 | 5.1 | 3.8 |

### Example 5

A crude fish oil was subjected to a separation and concentration process. A falling-film evaporator with a rectification column followed by a short path distillation was evaluated. The column contained approximately 2 meter of packing and had a mid-column liquid distributor. A marine oil was fed through the recycle stream at a temperature of 170°C. Concentration results are shown in Table 9. Isomer results are shown in Table 10.

**Table 9: Column Followed by Short Path Distillation, 2m packing, EPA/DHA concentration**

| **Sample** | **EPA (mg/g)** | **DHA (mg/g)** |
|---|---|---|
| Starting oil | 175.7 | 85 |
| Column-Distillate | 15.0 | 0.0 |
| Column-Residue | 361.1 | 177.7 |
| SPD-Distillate | 382.4 | 184.2 |
| SPD-Residue | 35.5 | 28.9 |

**Table 10: Column Followed by Short Path Distillation, 2m packing, Isomers**

| **Sample** | **EPA Isomers (%)** | **DHA Isomers (%)** | **Total Isomers (%)** |
|---|---|---|---|
| Starting oil | 0.3 | 0.8 | 0.5 |
| Column-Distillate | 3.7 | n/a | 3.7 |
| Column-Residue | 3.0 | 6.7 | 4.9 |
| SPD-Distillate | 2.1 | 5.7 | 4.1 |

### Example 6

A crude fish oil was subjected to a separation and concentration process. 2 columns in series (each a falling-film evaporator with a rectification column) followed by short path distillation was evaluated. The column-residue sample in Example 4 was fed through a second falling-film evaporator with a rectification column having approximately 2 meter of packing and a mid-column liquid distributor under the same conditions as in Example 4. Concentration results are shown in Table 11. Isomer results are shown in Table 12.

**Table 11: Column Followed by Short Path Distillation, 2m packing, EPA/DHA concentration**

| **Sample** | **EPA (mg/g)** | **DHA (mg/g)** |
|---|---|---|
| Starting oil | 175.7 | 85 |
| Column 1-Distillate | 15.0 | 0.0 |
| Column 1-Residue | 361.1 | 177.7 |
| Column 2-Distillate | 653.1 | 20.8 |
| Column 2-Residue | 0.0 | 145.5 |
| SPD-Distillate | 2.1 | 227.5 |
| SPD-Residue | 0.0 | 10.7 |

**Table 12: Column Followed by Short Path Distillation, 2m packing, Isomers**

| **Sample** | **EPA Isomers (%)** | **DHA Isomers (%)** | **Total Isomers (%)** |
|---|---|---|---|
| Starting oil | 0.3 | 0.8 | 0.5 |
| Column 1-Distillate | 3.7 | n/a | 3.7 |
| Column 1-Residue | 3.0 | 6.7 | 4.9 |
| Column 2-Distillate | 0.8 | 3.3 | 1.1 |
| Column 2-Residue | 6.6 | 24.4 | 23.6 |
| SPD-Distillate | 17.6 | 29.4 | 29.2 |

### Example 7

A crude fish oil was subjected to a separation and concentration process. A fractional distillation column with an external condenser followed by short path distillation (SPD) was evaluated. The external condenser was a wiped-film evaporator. The column was packed with 3 meters of packing, having approximately 8-9 theoretical stages of separation with a mid-column feed. Concentration results are shown in Table 13. Isomer results are shown in Table 14.

**Table 13: Column followed by Short-Path Distillation, 3m packing, EPA/DHA concentration**

| **Sample** | **EPA (mg/g)** | **DHA (mg/g)** |
|---|---|---|
| Starting oil | 173 | 88.2 |
| Column 1-Distillate | 13 | 0.0 |
| Column 1-Residue | 402.2 | 206.2 |
| SPD-Distillate | 450.3 | 228.1 |
| SPD-Residue | 56.9 | 51.7 |

**Table 14: Column followed by Short-Path Distillation, 3m packing, Isomers**

| **Sample** | **EPA Isomers (%)** | **DHA Isomers (%)** | **Total Isomers (%)** |
|---|---|---|---|
| Starting oil | 0.9 | 1.2 | 1.0 |
| Column 1-Residue | 0.6 | 0.7 | 0.6 |
| SPD-Distillate | 0.5 | 0.4 | 0.5 |

### Example 8

A crude fish oil was subjected to a separation and concentration process. A fractional distillation column with an external condenser followed by short path distillation (SPD) was evaluated. The external condenser was a wiped-film evaporator. The column was packed with 3 meters of packing, having approximately 8-9 theoretical stages of separation with an evaporator feed. Concentration results are shown in Table 15. Isomer results are shown in Table 16.

**Table 15: Column followed by Short-Path Distillation, 3m packing, EPA/DHA concentration**

| **Sample** | **EPA (mg/g)** | **DHA (mg/g)** |
|---|---|---|
| Starting oil | 173 | 88.2 |
| Column 1-Distillate | 11 | 0.0 |
| Column 1-Residue | 409 | 224.3 |

**Table 16: Column followed by Short-Path Distillation, 3m packing, Isomers**

| **Sample** | **EPA Isomers (%)** | **DHA Isomers (%)** | **Total Isomers (%)** |
|---|---|---|---|
| Starting oil | 0.9 | 1.2 | 1.0 |
| Column 1-Residue | 0.3 | 0.6 | 0.5 |

### Example 9

A crude fish oil was subjected to a separation and concentration process. A fractional distillation column with an external condenser followed by a second fractional distillation column with an external condenser followed by a short path distillation (SPD) was evaluated. The external condenser was a wiped-film evaporator. Both columns were packed with 2 meters of packing, having approximately 8-9 theoretical stages of separation with mid-column feed. Concentration results are shown in Table 17. Isomer results are shown in Table 18.

**Table 17: Column followed by Second Column, followed by Short-Path Distillation, 2m packing, EPA/DHA concentration**

| **Sample** | **EPA (mg/g)** | **DHA (mg/g)** |
|---|---|---|
| Starting oil | 175.2 | 83.7 |
| Column 1-Distillate | 15.0 | 0.0 |
| Column 1-Residue | 402.2 | 206.2 |
| Column 2-Feed | 407 | 211.1 |
| Column 2-Distillate | 825.1 | 0.0 |
| Column 2-Residue | 33 | 396.6 |
| SPD-Distillate | 21.9 | 493.2 |
| SPD-Residue | 0.0 | 64.4 |

**Table 18: Column followed by Second Column followed by Short-Path Distillation, 2m packing, Isomers**

| **Sample** | **EPA Isomers (%)** | **DHA Isomers (%)** | **Total Isomers (%)** |
|---|---|---|---|
| Column 2-Feed | 0.5 | 0.6 | 0.6 |
| Column 2-Distillate | 0.3 | 0.0 | 0.3 |
| Column 2-Distillate | 0.8 | 1.0 | 1.0 |
| SPD-Distillate | 4.4 | 0.5 | 0.6 |

### Example 10

Crude fish oils of various concentrations were subjected to a separation and concentration process. The starting oil concentrations listed in Tables 19-21 are representative concentration values based on the type of fish used (for example, 22:08 is a fish containing approximately 20% EPA and 8% DHA). A fractional distillation column with an external condenser followed by a second fractional distillation column with an external condenser followed by a short path distillation (SPD) was evaluated. The external condenser was a wiped-film evaporator. After passing through the first column, the EPA and DHA concentrations in the residue were measured (Table 19). The residue was passed through a second column and the EPA and DHA concentrations in the distillate were measured (Table 20). The residue from the second column was passed through a short path distillation column and the EPA and DHA concentrations in the residue were measured (Table 21).

**Table 19: First Column, EPA/DHA concentration**

| **Sample** | **Starting Material** | **Starting EPA (wt%)** | **Starting DHA (wt%)** | **Residue EPA (wt%)** | **Residue DHA (wt%)** |
|---|---|---|---|---|---|
| 1 | 22:8 | 17.5 | 8.4 | 39.5-42.8 | 20.6-21.8 |
| 2 | 22:8 | 17.5 | 8.4 | 40.5-41.5 | 21.0-21.2 |
| 3 | 18:12 | 14.3 | 12.6 | 29.5-32.4 | 29.9-31.6 |
| 4 | 18:12 | 14.3 | 12.6 | 30.4-32.6 | 29.8-30.8 |
| 5 | 22:8 | 17.5 | 8.4 | 40.6-41.0 | 21.1 |
| 6 | 12:18 | 13.9 | 13.0 | 28.7-31.7 | 30.8-32.4 |
| 7 | 12:18 | 13.9 | 13.0 | 28.5-31.8 | 30.4-32.4 |

**Table 20: Second Column, EPA/DHA concentration, Distillate**

| **Sample** | **Starting Material** | **Starting EPA (wt%)** | **Starting DHA (wt%)** | **Distillate EPA (wt%)** | **Distillate DHA (wt%)** |
|---|---|---|---|---|---|
| 1 | 22:8 | 40.7 | 21.1 | 79.8-82.2 | 0.0 |
| 2 | 18:12 | 31.1 | 30.6 | 75.5-78.8 | 0.0-0.04 |
| 3 | 12:18 | 30.6 | 31.3 | 74.4-76.0 | 0.0 |
| 4 | 12:18 | 30.6 | 31.3 | 73.9-74.6 | 0.0 |
| 5 | 22:8 | 39.9 | 20.6 | 75.1-77.8 | 0.0-0.66 |

**Table 21: Short Path Distillation Column, EPA/DHA concentration, Residue**

| **Sample** | **Starting Material** | **Starting EPA (wt%)** | **Starting DHA (wt%)** | **Residue EPA (wt%)** | **Residue DHA (wt%)** |
|---|---|---|---|---|---|
| 1 | 22:8 | 1.8 | 41.0 | 2.1-2.2 | 48.5-50.1 |
| 2 | 18:12 | 0.3 | 50.0 | 0.3-0.5 | 59.6-59.9 |
| 3 | 12:18 | 0.2 | 50.7 | 0.2 | 60.0 |

### Example 11

Crude fish oil having an EPA:DHA profile of 14.9 wt% EPA and 10.1 wt% DHA was subjected to a separation and concentration process. A fractional distillation column with an external condenser followed by a second fractional distillation column with an external condenser followed by a short path distillation (SPD) was evaluated. The external condenser was a wiped-film evaporator. After passing through the first column, the EPA and DHA concentrations in the distillate and residue were measured (Table 22). The residue was passed through a second column and the EPA and DHA concentrations in the distillate and residue were measured (Table 23). The residue from the second column was passed through a short path distillation column and the EPA and DHA concentrations in the distillate and residue were measured (Table 24). The EPA and DHA concentrations of each sample were measured at different time intervals during the processing.

**Table 22: First Column, EPA/DHA concentration**

| **Sample** | **Starting EPA (wt%)** | **Starting DHA (wt%)** | **Distillate EPA (wt%)** | **Distillate DHA (wt%)** | **Residue EPA (wt%)** | **Residue DHA (wt%)** |
|---|---|---|---|---|---|---|
| 1 | 14.9 | 10.1 | 5.8 | --- | 30.2 | 27.6 |
| 2 | 14.9 | 10.1 | 2.4 | --- | 33.7 | 26.0 |
| 3 | 14.9 | 10.1 | 1.9 | --- | 33.9 | 25.7 |
| 4 | 14.9 | 10.1 | 0.2 | --- | 35.0 | 24.4 |
| 5 | 14.9 | 10.1 | 0.2 | --- | 34.8 | 24.4 |

**Table 23: Second Column, EPA/DHA concentration**

| **Sample** | **Feed EPA (wt%)** | **Feed DHA (wt%)** | **Distillate EPA (wt%)** | **Distillate DHA (wt%)** | **Residue EPA (wt%)** | **Residue DHA (wt%)** |
|---|---|---|---|---|---|---|
| 1 | 35.6 | 24.9 | 61.3 | 0.1 | --- | 46.8 |
| 2 | 35.6 | 24.9 | 73.9 | --- | --- | 46.2 |
| 3 | 35.6 | 24.9 | 73.3 | 0.3 | --- | 46.3 |
| 4 | 35.6 | 24.9 | 74.0 | --- | 0.2 | 46.0 |
| 5 | 35.6 | 24.9 | 76.4 | --- | 0.3 | 45.5 |
| 6 | 35.6 | 24.9 | 76.7 | --- | 0.2 | 45.2 |
| 7 | 35.6 | 24.9 | 77.5 | --- | 0.3 | 44.2 |
| 8 | 35.6 | 24.9 | 77.0 | --- | 0.3 | 44.8 |

**Table 24: Short Path Distillation Column, EPA/DHA concentration**

| **Sample** | **Feed EPA (wt%)** | **Feed DHA (wt%)** | **Distillate EPA (wt%)** | **Distillate DHA (wt%)** | **Residue EPA (wt%)** | **Residue DHA (wt%)** |
|---|---|---|---|---|---|---|
| 1 | 0.2 | 45.5 | 0.2 | 55.9 | --- | 0.5 |
| 2 | 0.2 | 45.5 | 0.2 | 57.5 | --- | 1 |
| 3 | 0.2 | 45.5 | 0.2 | 56.5 | --- | 1.1 |
| 4 | 0.2 | 45.5 | 0.2 | 56.6 | --- | 2.3 |
| 5 | 0.2 | 45.5 | 0.2 | 56.7 | --- | 2.6 |
| 6 | 0.2 | 45.5 | 0.2 | 57 | --- | 3.3 |
| 7 | 0.2 | 45.5 | 0.2 | 58.4 | --- | 1.2 |

### Example 12

Crude fish oil was subjected to a separation and concentration process. A fractional distillation column with an external condenser followed by a second fractional distillation column with an external condenser followed by a short path distillation (SPD) was evaluated. The external condenser was a wiped-film evaporator. The levels of isomers were measured in these samples. Results are shown in Table 25.

**Table 25: Isomer Results**

| **Sample** | **Isomer A** | **Isomer B** | **Isomer C** | **Isomer D + E** | **Total Isomers** |
|---|---|---|---|---|---|
| Starting Oil (18:12 Fish Oil) | 0.26 | 0.07 | 0.12 | 0.34 | 0.79 |
| Distillate, First column | 0.0 | 0.0 | 0.0 | 0.0 | 0.0 |
| Residue, First column | 0.29 | 0.12 | 0.11 | 0.45 | 0.96 |
| Distillate, Second Column (~77 wt% EPA) | 0.33 | 0.16 | 0.16 | 0.51 | 1.16 |
| Distillate, Short-Path Distillation Column (~56 wt% DHA) | No or trace | No or trace | No or trace | No or trace | No or trace |

### Example 13

A crude oil was produced through fermentation of *Schizochytrium sp.* The oil was subjected to a transesterification step to produce ethyl esters of polyunsaturated fatty acids.

*Purification and Concentration:* A fractional distillation column with an external condenser followed by a short path distillation column was evaluated. The external condenser was a wiped-film evaporator. The fatty acid profile of the starting oil is shown in Table 26. The fatty acid profile of the oil produced is shown in Table 27.

**Table 26: Fatty acid profile, starting oil**

| **Fatty Acid** | **Weight Percent (%)** |
|---|---|
| C14:0 | 1.5 |
| C16:0 | 22.9 |
| C18:0 | 1.7 |
| C18:1 n-9 | 2.1 |
| C20:4 n-6 | 1.7 |
| C20:5 n-3 (EPA) | 19.9 |
| C22:5 n-6 | 1.9 |
| C22:5 n-3 | 3.3 |
| C22:6 n-3 (DHA) | 41.4 |

**Table 27: Fatty acid profile, oil**

| **Fatty Acid** | **Weight Percent (%)** |
|---|---|
| C16:0 | 12.3 |
| C18:0 | 1.5 |
| C18:1 n-9 | 1.9 |
| C20:4 n-6 | 1.8 |
| C20:5 n-3 (EPA) | 21.8 |
| C22:5 n-6 | 2.4 |
| C22:5 n-3 | 4.1 |
| C22:6 n-3 (DHA) | 50.6 |

### Example 14

A crude oil was produced through fermentation of *Schizochytrium sp.* The oil was subjected to a transesterification step to produce ethyl esters of polyunsaturated fatty acids.

*Purification and Concentration:* The oil was fractionated via a first pass through a fractional distillation column. The distillate contained 222 mg/g EPA and 218 mg/g DHA (sample 1D). The residue contained 175 mg/g EPA and 611 mg/g DHA (sample 1R). The distillate from this first pass was fractionated via a second pass through a fractional distillation column. The distillate from this was 77 mg/g EPA and 34 mg/g DHA (sample 3D) and the residue from this was 291 mg/g EPA and 331 mg/g DHA (sample 3R). The residue from the first pass was fractionated via a second pass through a fractional distillation column. The distillate from this was 181 mg/g EPA and 624 mg/g DHA (sample 2D) and the residue from this could not be sampled due to viscosity. Sample 2D was passed through a short path distillation apparatus. The distillate from this was 256 mg/g EPA and 559 mg/g DHA (sample 4D) and the residue was 105 mg/g EPA and 697 mg/g DHA (sample 4R). All passes through the fractionation column and/or short path distillation apparatus used a temperature range from 110°C to 180°C and vacuum of approximately 20-25mtorr. Results are shown in Table 28.

**Table 28: EPA/DHA concentration**

| **Sample** | **EPA (mg/g)** | **EPA (wt%)** | **DHA (mg/g)** | **DHA (wt%)** | **Total omega-3 (mg/g)** | **Total omega-3 (wt%)** |
|---|---|---|---|---|---|---|
| Starting oil | 193 | 19.3 | 392 | 39.2 | 624 | 62.4 |
| Column 1-Distillate (Sample 1D) | 222 | 22.2 | 218 | 21.8 | 465 | 46.5 |
| Column 1-Residue (Sample 1R) | 175 | 17.5 | 611 | 61.1 | 842 | 84.2 |
| Column 2-Distillate (Sample 2D) | 181 | 18.1 | 624 | 62.4 | 864 | 86.4 |
| Column 2-Residue (Sample 2R) | - | - | - | - | - | - |
| Column 3-Distillate (Sample 3D) | 77 | 7.7 | 34 | 3.4 | 116 | 11.6 |
| Column 3-Residue (Sample 3R) | 291 | 29.1 | 331 | 33.1 | 650 | 65.0 |
| SPD-Distillate (Sample 4D) | 256 | 25.6 | 559 | 55.9 | 857 | 85.7 |
| SPD-Residue (Sample 4R) | 105 | 10.5 | 697 | 69.7 | 860 | 86.0 |

### Example 15

crude oil was produced through fermentation of *Cryptochodinium cohnii.* The oil was subjected to a transesterification step to produce ethyl esters of polyunsaturated fatty acids.

*Purification and Concentration:* A fractional distillation column with an external condenser followed by a short path distillation was evaluated. The external condenser was a wiped-film evaporator. The fatty acid profile of the starting oil is shown in Table 29. Two trials were completed. The fatty acid profile of the oils produced are shown in Tables 30 and 31.

**Table 29: Fatty acid profile, starting oil**

| **Fatty Acid** | **Weight Percent (%)** |
|---|---|
| C12:0 | 5.09 |
| C14:0 | 9.10 |
| C16:0 | 6.74 |
| C18:1 n-9 | 6.42 |
| C20:5 n-3 (EPA) | 0.0 |
| C22:5 n-6 | 0.0 |
| C22:5 n-3 | 0.87 |
| C22:6 n-3 (DHA) | 48.7 |

**Table 30: Fatty acid profile, oil**

| **Fatty Acid** | **Weight Percent (%)** |
|---|---|
| C12:0 | 0.0 |
| C14:0 | 0.0 |
| C16:0 | 0.0 |
| C18:1 n-9 | 0.0 |
| C20:5 n-3 (EPA) | 0.0 |
| C22:5 n-6 | 0.0 |
| C22:5 n-3 | 1.7 |
| C22:6 n-3 (DHA) | 96.9 |

**Table 31: Fatty acid profile, oil**

| **Fatty Acid** | **Weight Percent (%)** |
|---|---|
| C12:0 | 0.0 |
| C14:0 | 0.0 |
| C16:0 | 0.0 |
| C18:1 n-9 | 0.0 |
| C20:5 n-3 (EPA) | 0.0 |
| C22:5 n-6 | 0.0 |
| C22:5 n-3 | 1.36 |
| C22:6 n-3 (DHA) | 96.6 |

## Claims

1. A process for separation and concentration of an oil comprising ethyl esters of polyunsaturated fatty acids, the process comprising subjecting the oil to at least one distillation step, said at least one distillation step comprising a first and a second distillation step, wherein the first distillation step comprises feeding the oil to at least one apparatus and subjecting the oil to conditions comprising heating to remove low-boiling compounds in a distillate and wherein the second distillation step comprises subjecting the oil to conditions to separate at least a portion of one long-chain polyunsaturated fatty acid (LC-PUFA) which is eicosapentaenoic acid (EPA) from at least a portion of a second LC-PUFA which is docosahexaenoic acid (DHA), wherein said at least one apparatus comprises a fractionating column that is attached to a wiped-film evaporator, wherein at least a portion of eicosapentaenoic acid (EPA) is removed in the distillate of the first distillation step, and wherein the oil has an isomer value of not more than 1% by weight, wherein the isomer value is determined using gas chromatography measurement, wherein the temperature during said at least one distillation step is less than about 275°C.

2. The process of claim 1, wherein a third distillation step comprises subjecting the oil to conditions to separate at least an additional portion of one long-chain polyunsaturated fatty acid (LC-PUFA) from a second LC-PUFA.

3. The process of any preceding claim, wherein the apparatus in a second distillation step comprises a short-path distillation column or a fractionating column.

4. The process of any preceding claim wherein at least a 5% of eicosapentaenoic acid (EPA) is removed in the distillate of the first distillation step, preferably wherein at least 10% EPA is removed in the distillate.

5. The process of any preceding claim, wherein at least one LC-PUFA is separated from a second LC-PUFA in an amount of at least about 50%.

6. The process of any preceding claim, wherein the wt% of at least one LC-PUFA is increased in the oil by at least about 20 wt% as compared to the wt% in the starting oil.

7. The process according to any preceding claim, wherein the oil comprises at least about 70% of a desired PUFA, preferably at least about 80% of a desired PUFA, more preferably at least about 90% of a desired PUFA.

8. The process according to any preceding claim, wherein the oil is a microbial or marine oil.

9. The process according to claim 8, wherein the oil is a microbial oil, preferably wherein the oil has been produced by a microorganism, wherein the microorganism is selected from the group comprising microalgae, bacteria, fungi and protists.

10. The process according to claim 9, wherein the oil has been produced by a microorganism, and wherein the microorganism is a microalgae.

11. The process according to claim 10, wherein the microalgae is of the genus *Thraustochytrium,* preferably wherein the microalgae is of the species *Schizochytrium sp.*

12. The process according to claim 9, wherein the microorganism is of the genus *Mortierella,* preferably wherein the microorganism is of the species *Mortierella alpina.*

13. The process according to any preceding claim, further comprising transesterifying the oil to convert at least part of the ester fraction in the oil to a triglyceride fraction.

14. An oil comprising an ester fraction, wherein the ester fraction comprises at least about 70% of the oil and wherein at least about 70% by weight of the fatty acids in the ester fraction is DHA in ethyl ester form and wherein
(i) from about 0.5% by weight to about 5% by weight of the fatty acids in the ester fraction is DPA n-3 in ethyl ester form and the amount of DHA in ethyl ester form is at least about 65% by weight of the total omega-3 fatty acids in the ester fraction; and
(ii) from about 3% to about 13% by weight of the fatty acids in the ester fraction is DPA n-3 in ethyl ester form and DPA n-6 in ethyl ester form and the amount of DHA in ethyl ester form is at least about 65% by weight of the total omega-3 fatty acids in the ester fraction; and
wherein the oil has an isomer value of not more than 1 wt%, wherein the isomer value is determined using gas chromatography measurement.

15. The oil of claim 14, wherein less than about 5% by weight of the fatty acids in the ester fraction is EPA in ethyl ester form, preferably wherein from about 0.1% to about 5% by weight of the fatty acids in the ester fraction is EPA in ethyl ester form.

16. The oil of any one of claims 14-15, wherein from about 1% to about 5% by weight of the fatty acids in the ester fraction is DPA n-3 in ethyl ester form.

17. The oil of any one of claims 14-16, wherein from about 2% to about 8% by weight of the fatty acids in the ester fraction is DPA n-6 in ethyl ester form.

18. The oil of any one of claims 14-17, wherein less than about 5% by weight of the fatty acids in the ester fraction is EPA in ethyl ester form, preferably wherein from about 0.1% to about 5% by weight of the fatty acids in the ester fraction is EPA in ethyl ester form.

19. The oil according to any one of claims 14-18, wherein the oil is a microbial or marine oil.

20. The oil according to any one of claims 14-19, wherein the oil is a microbial oil, wherein the oil has been produced by a microorganism, and wherein the microorganism is selected from the group comprising microalgae, bacteria, fungi and protists.

21. The oil according to claim 20, wherein the microorganism is a microalgae.

22. The oil according to claim 21, wherein the microalgae is of the genus *Thraustochytrium,* preferably wherein the microalgae is of the species *Schizochytrium sp.*

23. The oil according to claim 20, wherein the microorganism is of the genus *Mortierella,* preferably wherein the microorganism is of the species *Mortierella alpina.*

24. The oil according to any one of claims 14-23, wherein the oil is transesterified to convert at least part of the ester fraction in the oil to a triglyceride fraction and/or wherein the oil is produced by a genetically modified organism.

25. An oil of any one of claims 14-24 produced through the process of any one of claims 1-13.

## Patentansprüche

1. Verfahren zur Trennung und Konzentration eines Ethylester von mehrfach ungesättigten Fettsäuren umfassenden Öls, wobei man bei dem Verfahren das Öl mindestens einem Destillationsschritt unterzieht, wobei der mindestens eine Destillationsschritt einen ersten und einen zweiten Destillationsschritt umfasst, wobei man bei dem ersten Destillationsschritt das Öl mindestens einer Vorrichtung zuführt und das Öl Bedingungen unterzieht, die das Erhitzen zum Entfernen von niedrigsiedenden Verbindungen in einem Destillat umfassen, und wobei man bei dem zweiten Destillationsschritt das Öl Bedingungen unterzieht, bei denen mindestens ein Teil einer langkettigen mehrfach ungesättigten Fettsäure (LC-PUFA), bei der es sich um Eicosapentaensäure (EPA) handelt, von mindestens einem Teil einer zweiten LC-PUFA, bei der es sich um Docosasasasasäure (DHA) handelt, trennt, wobei die mindestens eine Vorrichtung eine Fraktionierkolonne umfasst, die mit einem Wischfilmverdampfer verbunden ist, wobei mindestens ein Teil der Eicosapentaensäure (EPA) im Destillat des ersten Destillationsschritts entfernt wird und wobei das Öl einen Isomerenwert von nicht mehr als 1 Gew.-% aufweist, wobei der Isomerenwert unter Anwendung einer Gaschromatographiemessung bestimmt wird, wobei die Temperatur während des mindestens einen Destillationsschritts weniger als etwa 275 °C beträgt.

2. Verfahren nach Anspruch 1, wobei man bei einem dritten Destillationsschritt das Öl Bedingungen aussetzt, bei denen mindestens einen zusätzlichen Teil einer langkettigen mehrfach ungesättigten Fettsäure (LC-PUFA) von einer zweiten LC-PUFA getrennt wird.

3. Verfahren nach einem der vorhergehenden Ansprüche, wobei die Vorrichtung in einem zweiten Destillationsschritt eine Kurzwegdestillationskolonne oder eine Fraktionierungskolonne umfasst.

4. Verfahren nach einem der vorhergehenden Ansprüche, wobei mindestens 5 % Eicosapentaensäure (EPA) in dem Destillat des ersten Destillationsschritts entfernt werden, wobei vorzugsweise mindestens 10 % EPA in dem Destillat entfernt werden.

5. Verfahren nach einem der vorhergehenden Ansprüche, wobei mindestens eine LC-PUFA von einer zweiten LC-PUFA in einer Menge von mindestens etwa 50 % getrennt wird.

6. Verfahren nach einem der vorhergehenden Ansprüche, wobei die Gew.-% mindestens einer LC-PUFA im Öl um mindestens etwa 20 Gew.-% im Vergleich zu den Gew.-% im Ausgangsöl erhöht werden.

7. Verfahren nach einem der vorhergehenden Ansprüche, wobei das Öl mindestens etwa 70 % einer gewünschten PUFA, bevorzugt mindestens etwa 80 % einer gewünschten PUFA, besonders bevorzugt mindestens etwa 90 % einer gewünschten PUFA umfasst.

8. Verfahren nach einem der vorhergehenden Ansprüche, wobei das Öl ein mikrobielles oder marines Öl ist.

9. Verfahren nach Anspruch 8, wobei das Öl ein mikrobielles Öl ist, wobei das Öl vorzugsweise von einem Mikroorganismus hergestellt wurde, wobei der Mikroorganismus aus der Mikroalgen, Bakterien, Pilze und Protisten umfassenden Gruppe ausgewählt ist.

10. Verfahren nach Anspruch 9, wobei das Öl von einem Mikroorganismus hergestellt wurde und wobei der Mikroorganismus eine Mikroalge ist.

11. Verfahren nach Anspruch 10, wobei die Mikroalgen der Gattung *Thraustochytrium* angehören, wobei die Mikroalgen vorzugsweise der Art *Schizochytrium sp.* angehören.

12. Verfahren nach Anspruch 9, wobei der Mikroorganismus der Gattung *Mortierella* angehört, wobei der Mikroorganismus vorzugsweise der Art *Mortierella alpina* angehört.

13. Verfahren nach einem der vorhergehenden Ansprüche, weiterhin umfassend eine Umesterung des Öls, bei der mindestens einen Teil der Esterfraktion in dem Öl in eine Triglyceridfraktion umgewandelt wird.

14. Öl, umfassend eine Esterfraktion, wobei die Esterfraktion mindestens etwa 70 % des Öls umfasst und wobei mindestens etwa 70 Gew.-% der Fettsäuren in der Esterfraktion DHA in Ethylesterform vorliegen und wobei
(i) etwa 0,5 Gew.-% bis etwa 5 Gew.-% der Fettsäuren in der Esterfraktion DPA n-3 in Ethylesterform sind und die Menge an DHA in Ethylesterform mindestens etwa 65 Gew.-% der gesamten Omega-3-Fettsäuren in der Esterfraktion beträgt und
(ii) etwa 3 Gew.-% bis etwa 13 Gew.-% der Fettsäuren in der Esterfraktion DPA n-3 in Ethylesterform und DPA n-6 in Ethylesterform sind und die Menge an DHA in Ethylesterform mindestens etwa 65 Gew.-% der gesamten Omega-3-Fettsäuren in der Esterfraktion beträgt und
wobei das Öl einen Isomerenwert von nicht mehr als 1 Gew.-% aufweist, wobei der Isomerenwert unter Anwendung einer Gaschromatographiemessung bestimmt wird.

15. Öl nach Anspruch 14, wobei weniger als etwa 5 Gew.-% der Fettsäuren in der Esterfraktion EPA in Ethylesterform vorliegen, wobei vorzugsweise etwa 0,1 Gew.-% bis etwa 5 Gew.-% der Fettsäuren in der Esterfraktion EPA in Ethylesterform vorliegen.

16. Öl nach einem der Ansprüche 14-15, wobei etwa 1 Gew.-% bis etwa 5 Gew.-% der Fettsäuren in der Esterfraktion DPA n-3 in Ethylesterform vorliegen.

17. Öl nach einem der Ansprüche 14-16, wobei etwa 2 Gew.-% bis etwa 8 Gew.-% der Fettsäuren in der Esterfraktion DPA n-6 in Ethylesterform vorliegen.

18. Öl nach einem der Ansprüche 14-17, wobei weniger als etwa 5 Gew.-% der Fettsäuren in der Esterfraktion EPA in Ethylesterform vorliegen, wobei vorzugsweise etwa 0,1 Gew.-% bis etwa 5 Gew.-% der Fettsäuren in der Esterfraktion EPA in Ethylesterform vorliegen.

19. Verfahren nach einem der Ansprüche 14 bis 18, wobei das Öl ein mikrobielles oder marines Öl ist.

20. Öl nach einem der Ansprüche 14-19, wobei das Öl ein mikrobielles Öl ist, wobei das Öl von einem Mikroorganismus produziert wurde und wobei der Mikroorganismus aus der Mikroalgen, Bakterien, Pilze und Protisten umfassenden Gruppe ausgewählt ist.

21. Öl nach Anspruch 20, wobei der Mikroorganismus eine Mikroalge ist.

22. Öl nach Anspruch 21, wobei die Mikroalgen der Gattung *Thraustochytrium* angehören, wobei die Mikroalgen vorzugsweise der Art *Schizochytrium* sp. angehören.

23. Öl nach Anspruch 20, wobei der Mikroorganismus der Gattung *Mortierella* angehört, wobei der Mikroorganismus vorzugsweise der Art *Mortierella alpina angehört.*

24. Öl nach einem der Ansprüche 14-23, wobei das Öl so umgeestert ist, dass mindestens ein Teil der Esterfraktion in dem Öl in eine Triglyceridfraktion umgewandelt ist, und/oder wobei das Öl von einem genetisch modifizierten Organismus produziert wird.

25. Öl nach einem der Ansprüche 14-24, hergestellt durch das Verfahren nach einem der Ansprüche 1-13.

## Revendications

1. Procédé de séparation et de concentration d'une huile comprenant des esters éthyliques d'acides gras polyinsaturés, le procédé comprenant la soumission de l'huile à au moins une étape de distillation, ladite au moins une étape de distillation comprenant une première et une deuxième étapes de distillation, dans lequel la première étape de distillation comprend l'alimentation de l'huile à au moins un appareil et la soumission de l'huile à des conditions comprenant le chauffage pour éliminer des composés à bas point d'ébullition dans un distillat et dans lequel la deuxième étape de distillation comprend la soumission de l'huile à des conditions pour séparer au moins une partie d'un acide gras polyinsaturé à longue chaîne (LC-PUFA) qui est l'acide eicosapentaénoïque (EPA) d'au moins une partie d'un second LC-PUFA qui est l'acide docosahexaénoïque (DHA), dans lequel ledit au moins un appareil comprend une colonne de fractionnement qui est fixée à un évaporateur à film essuyé, dans lequel au moins une partie de l'acide eicosapentaénoïque (EPA) est éliminée dans le distillat de la première étape de distillation, et dans lequel l'huile a une valeur d'isomère non supérieure à 1 % en poids, dans lequel la valeur d'isomère est déterminée par chromatographie en phase gazeuse, dans lequel la température pendant ladite au moins une étape de distillation est inférieure à environ 275 °C.

2. Procédé selon la revendication 1, dans lequel une troisième étape de distillation comprend la soumission de l'huile à des conditions pour séparer au moins une partie supplémentaire d'un acide gras polyinsaturé à longue chaîne (LC-PUFA) d'un second LC-PUFA.

3. Procédé selon l'une quelconque des revendications précédentes, dans lequel l'appareil dans une deuxième étape de distillation comprend une colonne de distillation à trajet court ou une colonne de fractionnement.

4. Procédé selon l'une quelconque des revendications précédentes, dans lequel au moins 5 % d'acide eicosapentaénoïque (EPA) sont éliminés dans le distillat de la première étape de distillation, de préférence dans lequel au moins 10 % d'EPA sont éliminés dans le distillat.

5. Procédé selon l'une quelconque des revendications précédentes, dans lequel au moins un LC-PUFA est séparé d'un second LC-PUFA en une quantité d'au moins environ 50 %.

6. Procédé selon l'une quelconque des revendications précédentes, dans lequel le pourcentage en poids d'au moins un LC-PUFA est augmenté dans l'huile d'au moins environ 20 % en poids par rapport au pourcentage en poids dans l'huile de départ.

7. Procédé selon l'une quelconque des revendications précédentes, dans lequel l'huile comprend au moins environ 70 % d'un PUFA souhaité, de préférence au moins environ 80 % d'un PUFA souhaité, plus préférablement au moins environ 90 % d'un PUFA souhaité.

8. Procédé selon l'une quelconque des revendications précédentes, dans lequel l'huile est une huile microbienne ou marine.

9. Procédé selon la revendication 8, dans lequel l'huile est une huile microbienne, de préférence dans lequel l'huile a été produite par un micro-organisme, dans lequel le micro-organisme est choisi dans le groupe comprenant les microalgues, les bactéries, les champignons et les protistes.

10. Procédé selon la revendication 9, dans lequel l'huile a été produite par un micro-organisme, et dans lequel le micro-organisme est une microalgue.

11. Procédé selon la revendication 10, dans lequel la microalgue est du genre *Thraustochytrium,* de préférence dans lequel la microalgue est de l'espèce *Schizochytrium* sp.

12. Procédé selon la revendication 9, dans lequel le micro-organisme est du genre *Mortierella,* de préférence dans lequel le micro-organisme est de l'espèce *Mortierella alpina.*

13. Procédé selon l'une quelconque des revendications précédentes, comprenant en outre la transestérification de l'huile pour convertir au moins une partie de la fraction ester dans l'huile en une fraction triglycéride.

14. Huile comprenant une fraction ester, dans laquelle la fraction ester comprend au moins environ 70 % de l'huile et dans laquelle au moins environ 70 % en poids des acides gras dans la fraction ester sont du DHA sous forme d'ester éthylique et dans laquelle
(i) entre environ 0,5 % en poids et environ 5 % en poids des acides gras dans la fraction ester sont du DPA n-3 sous forme d'ester éthylique et la quantité de DHA sous forme d'ester éthylique est d'au moins environ 65 % en poids des acides gras oméga-3 totaux dans la fraction ester ; et
(ii) environ 3 % à environ 13 % en poids des acides gras dans la fraction ester sont du DPA n-3 sous forme d'ester éthylique et DPA n-6 sous forme d'ester éthylique et la quantité de DHA sous forme d'ester éthylique est d'au moins environ 65 % en poids des acides gras oméga-3 totaux dans la fraction ester ; et
dans laquelle l'huile a une valeur d'isomère non supérieure à 1 % en poids, la valeur d'isomère étant déterminée par chromatographie en phase gazeuse.

15. Huile selon la revendication 14, dans laquelle moins d'environ 5 % en poids des acides gras dans la fraction ester sont de l'EPA sous forme d'ester éthylique, de préférence dans laquelle environ 0,1 % à environ 5 % en poids des acides gras dans la fraction ester sont de l'EPA sous forme d'ester éthylique.

16. Huile selon l'une quelconque des revendications 14 à 15, dans laquelle environ 1 % à environ 5 % en poids des acides gras dans la fraction ester sont du DPA n-3 sous forme d'ester éthylique.

17. Huile selon l'une quelconque des revendications 14 à 16, dans laquelle environ 2 % à environ 8 % en poids des acides gras dans la fraction ester sont du DPA n-6 sous forme d'ester éthylique.

18. Huile selon l'une quelconque des revendications 14 à 17, dans laquelle moins d'environ 5 % en poids des acides gras dans la fraction ester sont de l'EPA sous forme d'ester éthylique, de préférence dans laquelle environ 0,1 % à environ 5 % en poids des acides gras dans la fraction ester sont de l'EPA sous forme d'ester éthylique.

19. Huile selon l'une quelconque des revendications 14 à 18, dans laquelle l'huile est une huile microbienne ou marine.

20. Huile selon l'une quelconque des revendications 14 à 19, dans laquelle l'huile est une huile microbienne, dans laquelle l'huile a été produite par un micro-organisme, et dans laquelle le micro-organisme est choisi dans le groupe comprenant les microalgues, les bactéries, les champignons et les protistes.

21. Huile selon la revendication 20, dans laquelle le micro-organisme est une microalgue.

22. Huile selon la revendication 21, dans laquelle la microalgue est du genre *Thraustochytrium,* de préférence dans laquelle la microalgue est de l'espèce *Schizochytrium sp.*

23. Huile selon la revendication 20, dans laquelle le micro-organisme est du genre *Mortierella,* de préférence dans laquelle le micro-organisme est de l'espèce *Mortierella alpina.*

24. Huile selon l'une quelconque des revendications 14 à 23, dans laquelle l'huile est transestérifiée pour convertir au moins une partie de la fraction ester dans l'huile en une fraction triglycéride et/ou dans laquelle l'huile est produite par un organisme génétiquement modifié.

25. Huile selon l'une quelconque des revendications 14-24 produite par le procédé selon l'une quelconque des revendications 1 à 13.
